# EUROPEAN PATENT SPECIFICATION

(11) **EP 4 057 964 B1**
(45) Date of publication and mention of the grant of the patent: **16.08.2023**
(21) Application number: 20821072.4
(22) Date of filing: 23.10.2020
(51) Int. Cl.: A61F 13/47, A61F 13/472, A61F 13/534, A61F 13/15, A61F 13/53

(54) **SANITARY NAPKIN**
DAMENBINDE
SERVIETTE HYGIÉNIQUE

(30) Priority: 14.11.2019 SI 201900224
(43) Date of publication of application: 21.09.2022
(73) Proprietor: TOSAMA Tovarna sanitetnega materiala d.o.o., 1230 Domzale (SI)
(72) Inventor: ZABRET, Andrej, 1218 Komenda (SI); BRNOT, Katarina, 4220 Skofja Loka (SI)
(74) Representative: Borstar, Dusan
(86) International application number: PCT/SI2020/000007
(87) International publication number: WO 2021/096435

(56) References cited:
- WO-A1-81/01358
- WO-A2-02/053376
- FR-A1- 2 555 895
- US-A1- 2018 153 746

## Description

The invention refers to a sanitary napkin, namely to an absorbent pad for external application to the human body. Pursuant to the International patent Classification such invention in the field of human necessities and hygiene belong to domain of absorbent pads or tampons for external application to the ant therefore belong to class A 61 F 13/15.

In this, the invention is rest on a problem, how to conceive a sanitary napkin suitable for absorbing of a pre-determined quantity of liquid substance, in particular of human secretions and in particular of menstrual fluid, which should be placeable onto the external surface of a human body, namely between the skin or mucosa and each disposable underwear, and which should assure accurate and sufficient absorptiveness comparable to other state of the art napkins by simultaneously uniformly distributing and retaining of each absorbed fluid and avoiding unpleasant perception of contacting a totally wet pad, and which should consist of materials, which are biologically degradable within a reasonable time period, and preferably of natural plant raw materials.

A sanitary napkin is disclosed in EP 0 726 751 B and consists of a liquid impermeable bottom layer, which is during the use faced away from the user and is in contact with the underwear of the user, an absorbent core, which is placed over said bottom layer, as well as of a liquid permeable top layer, which is adapted for being placed onto skin or mucosa of the human body. Said absorbent core having a pre-determined density and weight per surface unit and is formed of non-woven fibers. To this aim, a mixture of natural and synthetic fibers on the basis of cotton, cellulose, polyester, polyamide or the like should be used. Said intermediate layer is intended for distribution of the liquid, which is absorbed within the absorbent core, over the complete area of the core, while said top layer should merely serve for ensuring pleasant feeling when contacting a skin or mucosa of the human body. Both said intermediate layer and top layer, in addition to natural fibers, also include synthetic fibers.

By such napkins a required absorption capacity, on which the frequency of replacing the napkin is depending, is no doubt assured, and moreover also a pleasant feeling of each user during the use thereof together with suitable contacting relationship with the underwear are also achieved, so that also uncontrolled displacing of the napkin during the use should be merely eliminated. The problem however arises after the use, when such napkin, saturated with body secretions, is thrown away. A liquid, which is absorbed i.e. contained within said absorbent core, is generally biologically degradable, which however does not apply for all ingredients of such napkin. If however in such napkin all artificial ingredients would be replaced with biologically degradable ingredients, then the absorption capacity would become insufficient, which would then from the point of view of each user lead to much more frequent replacing of a napkin, which is not preferred and might sometimes be unpleasant, and is less desired even from quite economic point of view. Another possible approach with the idea of applying more absorbent layers would lead to essential enlargement of thickness of the napkin, which would however not be accepted by the majority of users, either due to unpleasant filing, when rest on the skin or mucosa of the user, or due to perception that using of such napkin should not be sufficiently discrete.

Moreover, a similar sanitary napkin in the context of the previously mentioned technical problem, is also disclosed in EP 1 344 512 A1. Still further absorbent sanitary products are also disclosed in WO 81/01358 A1, FR 2 555 895 A1, US 2018/153746 A1 and WO 02/053376 A2.

The present invention refers to a sanitary napkin, which generally comprises a liquid impermeable bottom layer, which consists of a biologically degradable material and is during the use faced away from the user and supposed for being in contact with the underwear of each user, a liquid permeable top layer, which consists of a biologically degradable material and is during the use faced towards the user and is adapted for being in contact with the skin or mucosa of each user, as well as an absorbent core, which is placed between both previously mentioned layers and is adapted for absorbing of a predetermined quantity of liquid, in particular of human excrements, in particular of menstrual liquid.

The invention provides that said absorbent core between said liquid impermeable bottom layer consisting of biologically degradable material and said liquid permeable top layer consisting of biologically degradable material is integrally conceived and consists of a longitudinal section of a band consisting of non-woven biologically degradable natural plant fibers and having a pre-determined weight per length unit, wherein said band section comprises a central area of a predetermined width as well as two lateral longitudinal areas of said band, which are each per se folded and placed over said central area. The width of the first lateral longitudinal area exceeds the width of the second lateral longitudinal area for a pre-determined dimension, so that said central area of the band of the absorbent core of the napkin is overlapped on the one hand with the second longitudinal area and on the other hand also with the first lateral longitudinal area, the terminal area of which is overlapping said second lateral longitudinal area for said predetermined width. When observed in the transversal direction of the hygienic napkin, said terminal area of the first lateral longitudinal area, by which said second lateral longitudinal area is overlapped, is at least approximately centrally arranged, so that on the surface of said liquid permeable second layer of the sanitary napkin a smoothly rounded and outwardly deflected area in the shape of a longitudinal and centrally arranged rib of a pre-determined width is formed, so that the width (B1) of said rib (14) on the liquid permeable layer (12) due to the presence of said terminal area (131') of the first longitudinal lateral area (131), by which the second longitudinal lateral area (132) is overlapped, is determined within the range 5 - 45% of the width (C) of the central area of the absorbent core (13).

In one of the embodiments of the invention, said terminal area of the first lateral longitudinal area is placed between the second lateral longitudinal area and the central area of the band of the absorbent core.

In a further embodiment of the invention, the terminal area of the first lateral longitudinal area is placed between the second lateral longitudinal area of the band of the absorbent core and the liquid permeable top layer of the sanitary napkin.

In a still further embodiment of the invention, the terminal area of the first lateral longitudinal area is placed between the second lateral longitudinal area of the band of the absorbent core and the liquid impermeable bottom layer of the sanitary napkin.

In a still further embodiment of the invention, the liquid impermeable bottom layer is a foil consisting of biologically degradable polymers on the basis of cellulose, corn or potato starch, lactic acid and polyhydroxyalkanoates and/or polyethylene (PE) or polypropylene (PP), said liquid permeable top layer consists of nonwoven fibrous material on the basis of cotton and/or viscose and/or polyethylene (PE) and/or polypropylene (PP) and/or bamboo fibers, while the absorbent core consists of natural cotton fibers and/or fibers on the basis of viscose and/or cellulose fibers and/or bamboo fibers.

The invention further provides that the material of said absorbent core further comprises 0,36 - 2,17 g/m² of super absorbent on the basis of biodegradable polymers, in particular cellulose.

The thickness of the napkin, namely a complete thickness of liquid impermeable bottom layer and liquid permeable top layer as well as of said absorbent core formed of folded and each other overlapping layers does not exceed 6 mm.

Said absorbent core is formed of a band consisting of nonwoven biologically degradable plant fibers and having the weight per surface area 50 - 250 g/m², and preferably around 120 g/m², while its average absorption capacity is at least approximately 7 - 15 g/g.

The width of the central area of the band of the sanitary napkin is 40 - 80 mm, while the thickness of the band as such is around 1,5 mm.

The width of said rib on the liquid permeable layer due to the presence of said terminal area, by means of which the second longitudinal lateral area is overlapped by the first longitudinal lateral area, is 5 - 45% of the width of the central area, preferably 10 - 20% of the width of the central area of the absorbent core.

Embodiments of the invention are explained in more detail in connection with the attached drawings, in which
- Fig. 1: is one of possible embodiments of a sanitary napkin, which is schematically presented in top view prior to use;
- Fig. 2: is a cross-section of said sanitary napkin in a plane I - I according to Fig. 1;
- Fig. 3: is an absorbent core of a sanitary napkin according to Figs 1 and 2, which is as such presented schematically and in isometric view.

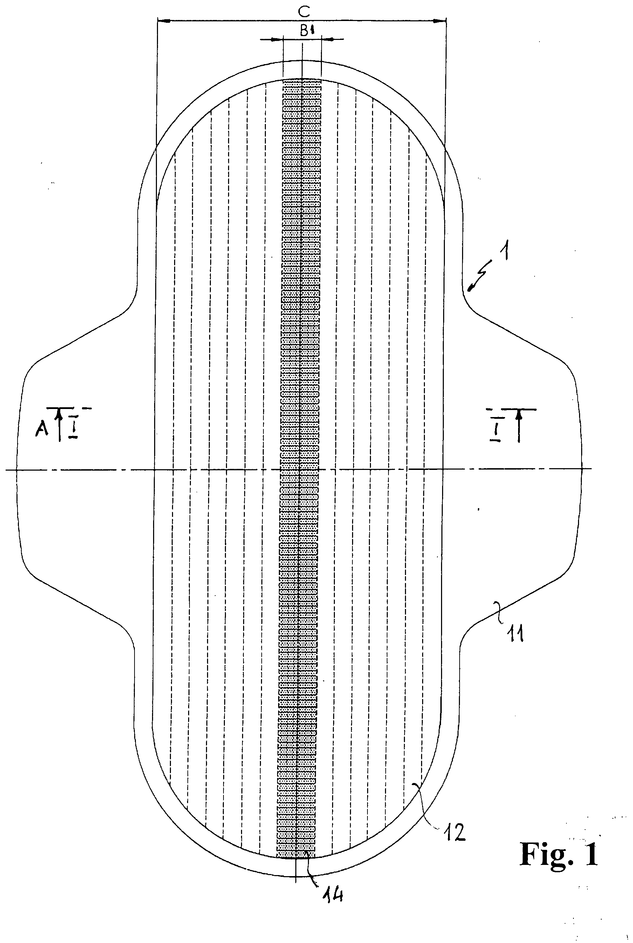

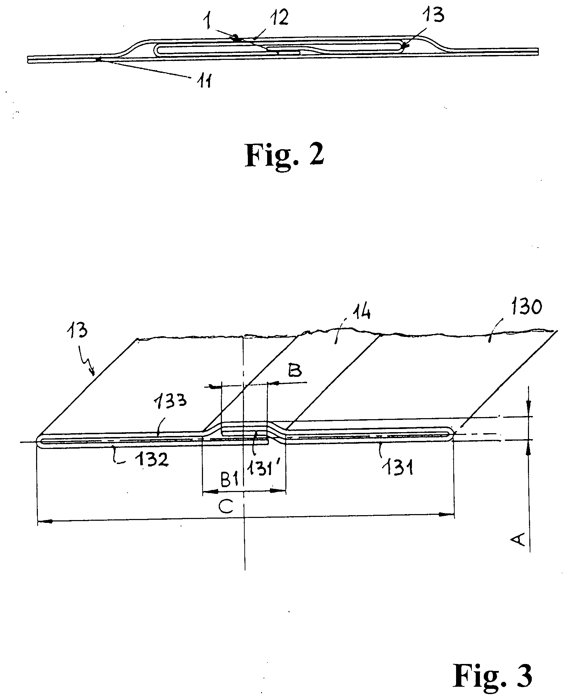

Sanitary napkin 1 according to Figs 1 and 2 comprises a liquid impermeable bottom layer 11, which consists of a biologically degradable material and is during the use faced away from the user and supposed for being in contact with the underwear of each user. In addition to said layer 11, said sanitary napkin 1 further comprises a liquid permeable top layer 12, which consists of a biologically degradable material and is during the use faced towards the user and is adapted for being in contact with the skin or mucosa of each user. Moreover, an absorbent core 13 is placed between both previously mentioned layers 11, 12 and is adapted for absorbing of a predetermined quantity of liquid, in particular of human secretions, in particular of menstrual fluid.

Said absorbent core 13 between said liquid impermeable bottom layer 11 consisting of biologically degradable material and said liquid permeable top layer 12 consisting of biologically degradable material is integrally conceived and, as such, consists of a longitudinal section of a band 130 consisting of non-woven biologically degradable natural plant fibers and having a pre-determined weight per length unit. Said band section comprises a central area 133 of a pre-determined width C as well as two lateral longitudinal areas 131, 132 of said band 130, which are each per se folded and placed over said central area 133. However, the width of the first lateral longitudinal area 131 exceeds the width of the second lateral longitudinal area 132 for a pre-determined dimension B, so that said central area 133 of the band 130 of the absorbent core 13 of the napkin 1 is overlapped on the one hand with the second longitudinal area 132 and on the other hand also with the first lateral longitudinal area 131, the terminal area 131' of which is overlapping said second lateral longitudinal area 132 for said pre-determined width B.

When observed in the transversal direction of the hygienic napkin 1, said terminal area 131' of the first lateral longitudinal area 131, by which said second lateral longitudinal area 132 is overlapped, is at least approximately centrally arranged, so that on the surface of said liquid permeable second layer 12 of the sanitary napkin 1 a smoothly rounded and outwardly deflected area in the shape of a longitudinal and centrally arranged rib 14 of a pre-determined width B1 is formed.

Thanks to the presence of said rib 14 on the top layer 12, which is during the use faced towards the user, such sanitary napkin 1 is ergonomically shaped and is adjusted to the shape of the user's body in the area where the napkin 1 is usually used. Moreover, due to the presence of said rib 14, in which in the area of absorption now there are three layers of the band 130 of the absorbent core 13 instead of just two layers, the absorption capacity and also the absorption rate are essentially improved, and in addition to that, each absorbed liquid is quickly efficiently transferred along the rib 14, namely both in a longitudinal direction of the sanitary napkin 1 as well as in both direction away from the rib 14 in the transversal direction of the sanitary napkin 1. Consequently, even by using exclusively biologically degradable fibers having some lower absorbing capacity than non-degradable synthetic fibers, the quantity of absorbed liquid is sufficient for the purpose of regular use, since such sanitary napkin 1 is thanks to its construction and shape suitable for retaining of each absorbed fluid at least to such extent that the user is prevented from unpleasant perception of being in contact with a totally wet napkin 1.

Several embodiments of such sanitary napkin 1 are foreseen in the context of overlapping of the second lateral longitudinal area 132 by said terminal area 131' of the first lateral longitudinal area 131. In the first embodiment, the terminal area 131' of the first lateral longitudinal area 131 is placed between the second lateral longitudinal area 132 and the central area 133 of the band 130 of the absorbent core 13. In the second embodiment, the terminal area 131' of the first lateral longitudinal area 131 is placed between the second lateral longitudinal area 132 of the band 130 of the absorbent core 13 and the liquid permeable top layer 12 of the sanitary napkin 1. In the third embodiment, the terminal area 131' of the first lateral longitudinal area 131 is placed between the second lateral longitudinal area 132 of the band 130 of the absorbent core 13 and the liquid impermeable bottom layer 11 of the sanitary napkin 1.

In these embodiments as discussed, said liquid impermeable bottom layer 11 is a foil consisting of biologically degradable polymers on the basis of cellulose, corn or potato starch, lactic acid and polyhydroxyalkanoates and/or polyethylene (PE) or polypropylene (PP), while said liquid permeable top layer 12 consists of nonwoven fibrous material on the basis of cotton and/or viscose and/or polyethylene (PE) and/or polypropylene (PP) and/or bamboo fibers. The absorbent core 13 consists of natural cotton fibers and/or fibers on the basis of viscose and/or cellulose fibers and/or bamboo fibers. In this, the material of said absorbent core 13 optionally comprises 0,36 - 2,17 g/m² of super absorbent on the basis of biologically degradable polymers, in particular cellulose. The thickness of such sanitary napkin 1, namely a complete thickness of liquid impermeable bottom layer 11 and liquid permeable top layer 12 as well as of said absorbent core 13 as formed of folded and each other overlapping layers 131, 131'; 132, 133, should not exceed 6 mm.

In a preferred embodiment of the sanitary napkin 1 according to the invention, said absorbent core 13 is formed of a band 130 consisting of nonwoven biologically degradable plant fibers and having the weight per surface area 50 - 250 g/m², and preferably around 120 g/m², while its average absorption capacity is at least approximately 7 - 15 g/g. The width C of the central area 133 of the band 130 of the sanitary napkin 1 should normally be 40 - 80 mm, while the thickness of the band 130 as such should be around 1,5 mm. The width B1 of said rib 14 on the liquid permeable layer 12 due to the presence of said terminal area 131' of the first longitudinal lateral area 131, by which the second longitudinal lateral area 132 is overlapped, is determined within the range 5 - 45% of the width C of the central area 133, preferably 10 - 20% of the width C of the central area 133 of the absorbent core 13.

## Claims

1. Sanitary napkin (1), comprising a liquid impermeable bottom layer (11), which consists of a biologically degradable material and is during the use faced away from the user and supposed for being in contact with the underwear of each user, a liquid permeable top layer (12), which consists of a biologically degradable material and is during the use faced towards the user and is adapted for being in contact with the skin or mucosa of each user, as well as an absorbent core (13), which is placed between both previously mentioned layers (11, 12) and is adapted for absorbing of a predetermined quantity of liquid, in particular of human excrements, in particular of menstrual liquid, **characterized in that** said absorbent core (13) between said liquid impermeable bottom layer (11) consisting of biologically degradable material and said liquid permeable top layer (12) consisting of biologically degradable material is integrally conceived and consists of a longitudinal section of a band (130) consisting of non-woven biologically degradable natural plant fibers and having a pre-determined weight per length unit, wherein said band section comprises a central area (133) of a pre-determined width (C) as well as two lateral longitudinal areas (131, 132) of said band (130), which are each per se folded and placed over said central area (133), wherein the width of the first lateral longitudinal area (131) exceeds the width of the second lateral longitudinal area (132) for a pre-determined dimension (B), so that said central area (133) of the band (130) of the absorbent core (13) of the napkin (1) is overlapped on the one hand with the second longitudinal area (132) and on the other hand also with the first lateral longitudinal area (131), the terminal area (131') of which is overlapping said second lateral longitudinal area (132) for said pre-determined width (B), and wherein, when observed in the transversal direction of the hygienic napkin (1), said terminal area (131') of the first lateral longitudinal area (131), by which said second lateral longitudinal area (132) is overlapped, is at least approximately centrally arranged, so that on the surface of said liquid permeable second layer (12) of the sanitary napkin (1) a smoothly rounded and outwardly deflected area in the shape of a longitudinal and centrally arranged rib (14) of a pre-determined width (B1) is formed, so that the width (B1) of said rib (14) on the liquid permeable layer (12) due to the presence of said terminal area (131') of the first longitudinal lateral area (131), by which the second longitudinal lateral area (132) is overlapped, is determined within the range 5 - 45% of the width (C) of the central area of the absorbent core (13).

2. Sanitary napkin according to Claim 1, **characterized in that** the terminal area (131') of the first lateral longitudinal area (131) is placed between the second lateral longitudinal area (132) and the central area (133) of the band (130) of the absorbent core (13).

3. Sanitary napkin according to Claim 1, **characterized in that** the terminal area (131') of the first lateral longitudinal area (131) is placed between the second lateral longitudinal area (132) of the band (130) of the absorbent core (13) and the liquid permeable top layer (12) of the sanitary napkin (1).

4. Sanitary napkin according to Claim 1, **characterized in that** the terminal area (131') of the first lateral longitudinal area (131) is placed between the second lateral longitudinal area (132) of the band (130) of the absorbent core (13) and the liquid impermeable bottom layer (11) of the sanitary napkin (1).

5. Sanitary napkin according to anyone of Claims 1-4, **characterized in that** said liquid impermeable bottom layer (11) is a foil consisting of biologically degradable polymers on the basis of cellulose, corn or potato starch, lactic acid and polyhydroxyalkanoates and/or polyethylene (PE) or polypropylene (PP), said liquid permeable top layer (12) consists of nonwoven fibrous material on the basis of cotton and/or viscose and/or polyethylene (PE) and/or polypropylene (PP) and/or bamboo fibers, while the absorbent core (13) consists of natural cotton fibers and/or fibers on the basis of viscose and/or cellulose fibers and/or bamboo fibers.

6. Sanitary napkin according to Claim 5, **characterized in that** the material of said absorbent core (13) further comprises 0,36 - 2,17 g/m² of super absorbent on the basis of biologically degradable polymers, in particular cellulose.

7. Sanitary napkin according to anyone of Claims 1-6, **characterized in that** the thickness of the napkin (1), namely a complete thickness of liquid impermeable bottom layer (11) and liquid permeable top layer (12) as well as of said absorbent core (13) as formed of folded and each other overlapping layers (131, 131'; 132, 133) does not exceed 6 mm.

8. Sanitary napkin according to anyone of Claims 1-6, **characterized in that** said absorbent core (13) is formed of a band (130) consisting of nonwoven biologically degradable plant fibers and having the weight per surface area 50 - 250 g/m², and preferably around 120 g/m², while its average absorption capacity is at least approximately 7 - 15 g/g.

9. Sanitary napkin according to Claim 8, **characterized in that** the width (C) of the central area (133) of the band (130) of the sanitary napkin (1) is 40 - 80 mm, while the thickness of the band (130) as such is around 1,5 mm.

10. Sanitary napkin according to anyone of Claims 1 - 9, **characterized in that** the width (B1) of said rib (14) on the liquid permeable layer (12) due to the presence of said terminal area (131') of the first longitudinal lateral area (131), by which the second longitudinal lateral area (132) is overlapped, is determined within the range 10 - 20% of the width (C) of the central area (133) of the absorbent core (13).

## Patentansprüche

1. Hygienebinde (1), umfassend: eine flüssigkeitsundurchlässige untere Schicht (11), die aus einem biologisch abbaubaren Material besteht und während des Gebrauchs dem Benutzer abgewandt ist und dafür bestimmt ist, in Kontakt mit der Unterwäsche jedes Benutzers zu stehen, eine flüssigkeitsdurchlässige obere Schicht (12), die aus einem biologisch abbaubaren Material besteht und während des Gebrauchs dem Benutzer zugewandt ist und dafür ausgelegt ist, in Kontakt mit der Haut oder Schleimhaut jedes Benutzers zu stehen, sowie einen absorbierenden Kern (13), der zwischen den beiden zuvor erwähnten Schichten (11, 12) angeordnet ist und dafür ausgelegt ist, eine zuvor festgelegte Menge an Flüssigkeit, insbesondere an menschlichen Ausscheidungen, insbesondere an Menstruationsflüssigkeit, zu absorbieren, **dadurch gekennzeichnet, dass** der absorbierende Kern (13) zwischen der flüssigkeitsundurchlässigen unteren Schicht (11), die aus biologisch abbaubarem Material besteht, und der flüssigkeitsdurchlässigen oberen Schicht (12), die aus biologisch abbaubarem Material besteht, integral konzipiert ist und aus einem Längsabschnitt eines Bandes (130) besteht, das aus einem biologisch abbaubaren natürlichen Pflanzenfaservlies besteht und ein zuvor festgelegtes Gewicht pro Längeneinheit aufweist, wobei der Bandabschnitt einen mittigen Bereich (133) mit einer zuvor festgelegten Breite (C) sowie zwei seitliche Längsbereiche (131, 132) des Bandes (130) umfasst, die jeweils für sich gefaltet und über dem mittigen Bereich (133) platziert sind, wobei die Breite des ersten seitlichen Längsbereichs (131) die Breite des zweiten seitlichen Längsbereichs (132) um eine zuvor festgelegte Abmessung (B) überschreitet, so dass der mittige Bereich (133) des Bands (130) des absorbierenden Kerns (13) der Binde (1) einerseits durch den zweiten Längsbereich (132) und andererseits auch durch den ersten seitlichen Längsbereich (131) überlappt wird, dessen Endbereich (131') den zweiten seitlichen Längsbereich (132) über die zuvor festgelegte Breite (B) überlappt, und wobei, in Querrichtung der Hygienebinde (1) gesehen, der Endbereich (131') des ersten seitlichen Längsbereichs (131), durch den der zweite seitliche Längsbereich (132) überlappt wird, mindestens ungefähr mittig angeordnet ist, so dass auf der Oberfläche der flüssigkeitsdurchlässigen zweiten Schicht (12) der Hygienebinde (1) ein sanft gerundeter und nach außen ausgelenkter Bereich in Form einer länglichen und mittig angeordneten Rippe (14) mit einer zuvor festgelegten Breite (B1) gebildet wird, so dass die Breite (B1) der Rippe (14) auf der flüssigkeitsdurchlässigen Schicht (12) aufgrund des Vorhandenseins des Endbereichs (131') des ersten seitlichen Längsbereichs (131), durch den der zweite seitliche Längsbereich (132) überlappt wird, innerhalb des Bereichs von 5-45 % der Breite (C) des mittigen Bereichs des absorbierenden Kerns (13) bestimmt wird.

2. Hygienebinde nach Anspruch 1, **dadurch gekennzeichnet, dass** der Endbereich (131') des ersten seitlichen Längsbereichs (131) zwischen dem zweiten seitlichen Längsbereich (132) und dem mittigen Bereich (133) des Bandes (130) des absorbierenden Kerns (13) platziert ist.

3. Hygienebinde nach Anspruch 1, **dadurch gekennzeichnet, dass** der Endbereich (131') des ersten seitlichen Längsbereichs (131) zwischen dem zweiten seitlichen Längsbereich (132) des Bandes (130) des absorbierenden Kerns (13) und der flüssigkeitsdurchlässigen oberen Schicht (12) der Hygienebinde (1) platziert ist.

4. Hygienebinde nach Anspruch 1, **dadurch gekennzeichnet, dass** der Endbereich (131') des ersten seitlichen Längsbereichs (131) zwischen dem zweiten seitlichen Längsbereich (132) des Bandes (130) des absorbierenden Kerns (13) und der flüssigkeitsundurchlässigen Bodenschicht (11) der Hygienebinde (1) platziert ist.

5. Hygienebinde nach einem der Ansprüche 1-4, **dadurch gekennzeichnet, dass** die flüssigkeitsundurchlässige untere Schicht (11) eine Folie ist, die aus biologisch abbaubaren Polymeren auf der Basis von Cellulose, Maisoder Kartoffelstärke, Milchsäure und Polyhydroxyalkanoaten und/oder Polyethylen (PE) oder Polypropylen (PP) besteht, die flüssigkeitsdurchlässige obere Schicht (12) aus Faservliesmaterial auf der Basis von Baumwolle und/oder Viskose und/oder Polyethylen (PE) und/oder Polypropylen (PP) und/oder Bambusfasern besteht, während der absorbierende Kern (13) aus natürlichen Baumwollfasern und/oder Fasern auf der Basis von Viskose und/oder Zellulosefasern und/oder Bambusfasern besteht.

6. Hygienebinde nach Anspruch 5, **dadurch gekennzeichnet, dass** das Material des absorbierenden Kerns (13) des Weiteren 0,36-2,17 g/m² eines Superabsorbens auf der Basis biologisch abbaubarer Polymere, insbesondere Zellulose, umfasst.

7. Hygienebinde nach einem der Ansprüche 1-6, **dadurch gekennzeichnet, dass** die Dicke der Binde (1), und zwar die vollständige Dicke der flüssigkeitsundurchlässigen unteren Schicht (11) und der flüssigkeitsdurchlässigen oberen Schicht (12) sowie des absorbierenden Kerns (13), die aus gefalteten und einander überlappenden Lagen (131, 131'; 132, 133) gebildet ist, 6 mm nicht überschreitet.

8. Hygienebinde nach einem der Ansprüche 1-6, **dadurch gekennzeichnet, dass** der absorbierende Kern (13) aus einem Band (130) gebildet ist, das aus biologisch abbaubarem Pflanzenfaservlies besteht und ein Flächengewicht von 50-250 g/m², bevorzugt ungefähr 120 g/m², aufweist, während seine durchschnittliche Absorptionskapazität mindestens ungefähr 7-15 g/g beträgt.

9. Hygienebinde nach Anspruch 8, **dadurch gekennzeichnet, dass** die Breite (C) des mittigen Bereichs (133) des Bandes (130) der Hygienebinde (1) 40-80 mm beträgt, während die Dicke des Bandes (130) als solches etwa 1,5 mm beträgt.

10. Hygienebinde nach einem der Ansprüche 1-9, **dadurch gekennzeichnet, dass** die Breite (B1) der Rippe (14) auf der flüssigkeitsdurchlässigen Schicht (12) aufgrund des Vorhandenseins des Endbereichs (131') des ersten seitlichen Längsbereichs (131), durch den der zweite seitlichen Längsbereichs (132) überlappt wird, innerhalb des Bereichs von 10-20 % der Breite (C) des mittigen Bereichs (133) des absorbierenden Kerns (13) bestimmt wird.

## Revendications

1. Serviette hygiénique (1), comprenant une couche de dessous imperméable aux liquides (11), laquelle est constituée d'un matériau biologiquement dégradable et, durant l'utilisation, est orientée à l'opposé de l'utilisateur et censée être en contact avec le sous-vêtement de chaque utilisateur, une couche de dessus perméable aux liquides (12), laquelle est constituée d'un matériau biologiquement dégradable et, durant l'utilisation, est orientée vers l'utilisateur et est adaptée à être en contact avec la peau ou la muqueuse de chaque utilisateur, ainsi qu'un coeur absorbant (13), lequel est placé entre les deux couches précédemment mentionnées (11, 12) et est adapté à l'absorption d'une quantité prédéterminée de liquide, en particulier d'excréments humains, en particulier de liquide menstruel, **caractérisée en ce que** ledit coeur absorbant (13) entre ladite couche de dessous imperméable aux liquides (11) constituée de matériau biologiquement dégradable et ladite couche de dessus perméable aux liquides (12) constituée de matériau biologiquement dégradable est conçu d'un seul tenant et est constitué d'une section longitudinale d'une bande (130) constituée de fibres végétales naturelles biologiquement dégradables non tissées et ayant un poids prédéterminé par unité de longueur, dans laquelle ladite section de bande comprend une zone centrale (133) d'une largeur prédéterminée (C) ainsi que deux zones longitudinales latérales (131, 132) de ladite bande (130), lesquelles sont chacune en soi pliées et placées par-dessus ladite zone centrale (133), dans laquelle la largeur de la première zone longitudinale latérale (131) dépasse la largeur de la deuxième zone longitudinale latérale (132) pour une dimension prédéterminée (B), de sorte que ladite zone centrale (133) de la bande (130) du coeur absorbant (13) de la serviette (1) soit chevauchée d'une part par la deuxième zone longitudinale (132) et d'autre part également par la première zone longitudinale latérale (131), dont la zone terminale (131') chevauche ladite deuxième zone longitudinale latérale (132) pour ladite largeur prédéterminée (B), et dans laquelle, lorsqu'elle est observée dans la direction transversale de la serviette hygiénique (1), ladite zone terminale (131') de la première zone longitudinale latérale (131), par laquelle est chevauchée ladite deuxième zone longitudinale latérale (132), est agencée de manière au moins approximativement centrale, de sorte que sur la surface de ladite deuxième couche perméable aux liquides (12) de la serviette hygiénique (1) soit formée une zone légèrement arrondie et déviée vers l'extérieur sous la forme d'une nervure longitudinale et agencée de manière centrale (14) d'une largeur prédéterminée (B1), de sorte que la largeur (B1) de ladite nervure (14) sur la couche perméable aux liquides (12) due à la présence de ladite zone terminale (131') de la première zone latérale longitudinale (131), par laquelle est chevauchée la deuxième zone latérale longitudinale (132), soit déterminée au sein de la gamme allant de 5 à 45 % de la largeur (C) de la zone centrale du coeur absorbant (13).

2. Serviette hygiénique selon la revendication 1, **caractérisée en ce que** la zone terminale (131') de la première zone longitudinale latérale (131) est placée entre la deuxième zone longitudinale latérale (132) et la zone centrale (133) de la bande (130) du coeur absorbant (13).

3. Serviette hygiénique selon la revendication 1, **caractérisée en ce que** la zone terminale (131') de la première zone longitudinale latérale (131) est placée entre la deuxième zone longitudinale latérale (132) de la bande (130) du coeur absorbant (13) et la couche de dessus perméable aux liquides (12) de la serviette hygiénique (1).

4. Serviette hygiénique selon la revendication 1, **caractérisée en ce que** la zone terminale (131') de la première zone longitudinale latérale (131) est placée entre la deuxième zone longitudinale latérale (132) de la bande (130) du coeur absorbant (13) et la couche de dessous imperméable aux liquides (11) de la serviette hygiénique (1).

5. Serviette hygiénique selon l'une quelconque des revendications 1 à 4, **caractérisée en ce que** ladite couche de dessous imperméable aux liquides (11) est une feuille constituée de polymères biologiquement dégradables à base de cellulose, d'amidon de maïs ou de pomme de terre, d'acide lactique et de polyhydroxyalcanoates et/ou de polyéthylène (PE) ou de polypropylène (PP), ladite couche de dessus perméable aux liquides (12) est constituée de matériau fibreux non tissé à base de fibres de coton et/ou de viscose et/ou de polyéthylène (PE) et/ou de polypropylène (PP) et/ou de bambou, tandis que le cœur absorbant (13) est constitué de fibres de coton naturel et/ou de fibres à base de fibres de viscose et/ou de cellulose et/ou de fibres de bambou.

6. Serviette hygiénique selon la revendication 5, **caractérisée en ce que** le matériau dudit coeur absorbant (13) comprend en outre de 0,36 à 2,17 g/m² de super absorbant à base de polymères biologiquement dégradables, en particulier de cellulose.

7. Serviette hygiénique selon l'une quelconque des revendications 1 à 6, **caractérisée en ce que** l'épaisseur de la serviette (1), à savoir une épaisseur complète de couche de dessous imperméable aux liquides (11) et de couche de dessus perméable aux liquides (12) ainsi que dudit coeur absorbant (13) tel que formé de couches pliées et se chevauchant les unes les autres (131, 131' ; 132, 133), ne dépasse pas 6 mm.

8. Serviette hygiénique selon l'une quelconque des revendications 1 à 6, **caractérisée en ce que** ledit coeur absorbant (13) est formé d'une bande (130) constituée de fibres végétales biologiquement dégradables non tissées et ayant le poids par unité de surface allant de 50 à 250 g/m², et de préférence autour de 120 g/m², tandis que sa capacité d'absorption moyenne va au moins approximativement de 7 à 15 g/g.

9. Serviette hygiénique selon la revendication 8, **caractérisée en ce que** la largeur (C) de la zone centrale (133) de la bande (130) de la serviette hygiénique (1) va de 40 à 80 mm, tandis que l'épaisseur de la bande (130) en tant que telle est autour de 1,5 mm.

10. Serviette hygiénique selon l'une quelconque des revendications 1 à 9, **caractérisée en ce que** la largeur (B1) de ladite nervure (14) sur la couche perméable aux liquides (12) due à la présence de ladite zone terminale (131') de la première zone latérale longitudinale (131), par laquelle est chevauchée la deuxième zone latérale longitudinale (132), est déterminée au sein de la gamme allant de 10 à 20 % de la largeur (C) de la zone centrale (133) du coeur absorbant (13).
